# EUROPEAN PATENT APPLICATION

(11) **EP 1 795 141 A2**
(43) Date of publication of application: **13.06.2007**
(21) Application number: 07005991.0
(22) Date of filing: 02.10.2003
(51) Int. Cl.: A61B 18/24

(54) **Thrombolysis cathether**

(30) Priority: 02.10.2002 US 262829
(62) Divisional of application: 03770618.1
(71) Applicant: Boston Scientific Limited, St. Michael, Barbados, West Indies (BB)
(72) Inventor: Couvillon, Lucien Alfred Jr., Concord, MA 01742 (US)
(74) Representative: Hauck Patent- und Rechtsanwälte

(57) **Abstract**

A thrombolysis catheter apparatus, comprising:
• an elongated thrombolysis catheter portion comprising a plurality of independently controllable electroactive polymer actuators, provided on substrate layer made from a flexible material and wrapped into a cylinder, said actuators providing a curvature for said thrombolysis catheter portion based upon received control signals;
• a control unit coupled to said plurality of actuators and sending said control signals to said plurality of actuators; and
• an occlusion removal device.

## Description

### STATEMENT OF RELATED APPLICATION

This patent application is related to U.S. Serial No. 09/971,419, filed October 5, 2001, to U.S. Serial No. 10/177,491, filed June 21, 2002, and to U.S. Serial No. 10/176,977, filed June 21, 2002.

### FIELD OF THE INVENTION

The present invention relates to thrombolysis catheters, and more particularly to thrombolysis catheters whose shape can be tailored to reflect the natural contours of a patient's blood vessels.

### BACKGROUND OF THE INVENTION

Occlusive stroke (also referred to as "brain attack") is a major public health problem caused by occlusion of the arteries of the brain. The occlusion can arise, for example, from a blood clot or embolus becoming lodged in one of the small arteries of the brain, and can lead to stroke, or even death, if left untreated. If recognized early enough, however, the occlusion can be removed by various techniques, including local or systemic administration of thrombolytic agents (e.g., heparin, urokinase, and so forth) as well as non-chemical techniques such as angioplasty, photoablative or elevated temperature thrombolysis (e.g., laser thrombolysis) and mechanical thrombolysis (e.g., hydraulic thrombolysis via fluid jet or ultrasound thrombolysis).

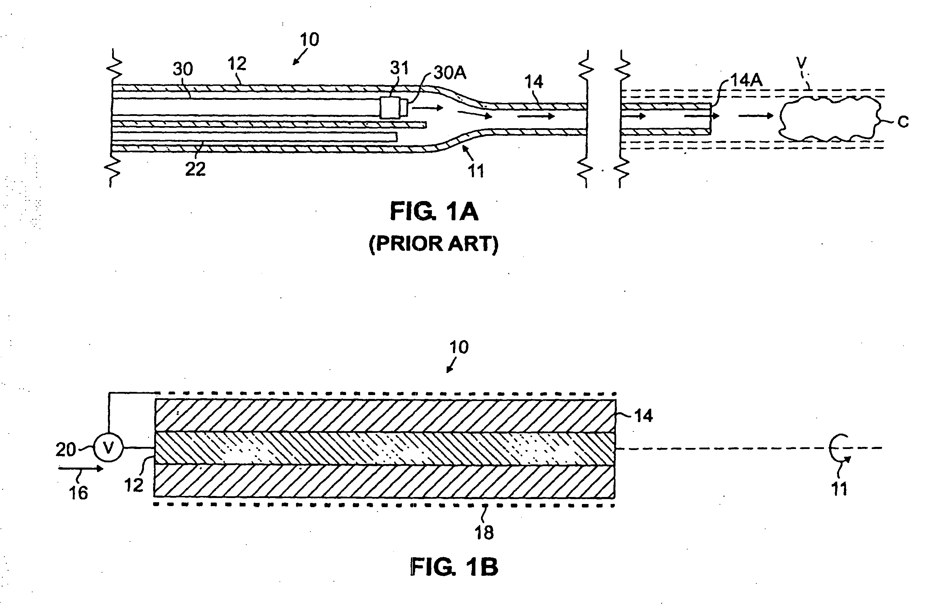

An example of a prior art thrombolysis catheter 10 can be found in Fig. 1A. The thrombolysis catheter 10 has a proximal portion 12 as well as a smaller, more flexible distal portion 14 for insertion into a lumen, such as a blood vessel V. The proximal portion 12 is provided with two internal lumens, one of which contains an optical fiber bundle 30, and the other of which contains a guidewire 22. The distal portion 14 is provided with a single lumen. Near the emission end 30A of the optical fiber bundle 30 is found a radio-opaque marker 31, which assists in achieving proper placement. During operation, the guidewire 22 is used to position the distal end 14A of the catheter 10 adjacent a selected site, such as clot C in vessel V. Once the catheter 10 is safely guided into the desired location in the body, the guidewire 22 can be withdrawn, whereupon a light-transmissive liquid (e.g. saline) is introduced into the catheter 10 and vessel V adjacent clot C. Laser energy is then launched from the emission end 30A of the fiber bundle 30 into the light transmissive liquid, which conveys the energy through the distal portion 14 of the catheter 10 to the clot C. The distal portion 14 of the catheter 10 can be provided, for example, with a sidewall that is capable of internally reflecting light, allowing the light-transmissive liquid to act as a waveguide for the laser energy emerging from the emission end of the fiber 30A. Upon emerging from the distal portion 14 of the catheter 10, the laser light strikes the clot C, removing the same from vessel V. Further information can be found in U.S. Patent No. 6,117,128, the entire disclosure of which is hereby incorporated by reference.

Unfortunately, localized thrombolysis procedures are presently hindered by the difficulties that are encountered in steering thrombolysis catheters like that described above through the vasculature, particularly the tortuous blood vessels of the neck and head, to the site of the occlusion. Because time is of the essence where occlusions of the neurovasculature are concerned, these difficulties have presented a major obstacle in the widespread acceptance of thrombolysis catheters in the treatment of occlusive stroke.

### SUMMARY OF THE INVENTION

The above and other challenges of the prior art are addressed by the novel thrombolysis catheter apparatus of the present invention, which comprises: (a) an elongated thrombolysis catheter portion comprising a plurality of independently controllable electroactive polymer actuators, in which the actuators providing a curvature to the thrombolysis catheter based upon received control signals; (b) a control unit coupled to the plurality of actuators and sending the control signals to the plurality of actuators; and (c) an occlusion removal device.

The above apparatus is useful in removing occlusions from the arterial vasculature of a patient. Typically, the thrombolysis catheter portion is advanced through the arterial vasculature of a patient, while using the control unit to control its shape. Once the site of the occlusion is reached, the occlusion removal device is used to remove the occlusion.

In many embodiments, the occlusion removal device comprises a laser and a waveguide, which allows laser light to be efficiently transmitted from a distal end of the thrombolysis catheter.

The control unit of the thrombolysis catheter apparatus of the present invention can comprise, for example, a computer, such as a personal computer or PDA (personal digital assistant) device. The control unit can be coupled to the actuators in a variety of ways, for example, via a multiplexed electrical cable or via a wireless interface. The electroactive polymer actuators are beneficially provided over a substantial portion of the thrombolysis catheter portion length. For example, the electroactive polymer actuators of the thrombolysis catheters of the present invention can be disposed over 5 cm, 10 cm, 15 cm, 20 cm, 25 cm, or more of the length of the thrombolysis catheter portion.

The electroactive polymer actuators can be controlled to provide a near infinite range of curvatures for the thrombolysis catheter portion, including in-plane curves (e.g., an "S" shaped curve) and out-of-plane curves (e.g., a helix) as well as other far more complex curvatures. For example, in some embodiments of the invention, the electroactive polymer actuators are controllable to impart an orientation to the thrombolysis catheter portion that is complementary to the natural orientation of the blood vessel(s) through which the thrombolysis catheter portion is advanced. For example, the thrombolysis catheter portion can be provided with an out-of-plane curvature that corresponds to the natural orientation of at least a portion of the arterial vasculature, for example, the natural orientation of at least a portion of a cranial artery or an internal carotid artery.

Complex shapes are generated using large numbers of electroactive polymer actuators in some embodiments, for example, 10, 25, 50, 100, 250, 500, 1000 or more actuator elements can be utilized, with increased numbers of actuator elements giving finer curvature detail.

In some embodiments of the invention, the control signals from the control unit are generated based on medical diagnostic imaging data, for example, imaging data generated from diagnostic angiograms, sonograms, CT (computed tomography) or MR (magnetic resonance) scans, IVUS (intravascular ultrasound) data, or fluoroscopic images (which may be multiplane or tomographic), or electromagnetic sensors provided within the catheter portion. In other embodiments, the control signals from the control unit are generated, for example, using a manual steering device.

In one embodiment, the thrombolysis catheter portion comprises a lead module and a plurality of following modules. In this configuration, when each following module reaches a position previously occupied by the lead module, the control system and actuators cause the following module to replicate the orientation that the lead module had when it was at that particular position. Module orientation data can be provided, for example, by strain gauges within each module. Position data can be provided, for example, by a depth measurement device, such as a depth gauge or a linear displacement module. Alternatively, position data can be provided using, for example, imaging data such as that described above, including data generated from diagnostic angiograms, sonograms, CT or MR scans, IVUS data, or fluoroscopic images, or radiographic data or data generated using electromagnetic position sensors within the catheter portion.

In certain embodiments, at least a portion of the actuators are in tension with one another. This allows, for example, for the thrombolysis catheter portion to be stiffened after reaching a desired location within the body, if desired.

The electroactive polymer actuators typically comprise (a) an active member, (b) a counter-electrode and (c) an electrolyte-containing region disposed between the active member portion and the counter-electrode portion. In some embodiments, the thrombolysis catheter portion will comprise a substrate layer, and the active member, the counter-electrode and the electrolyte-containing region will be disposed over the substrate layer. In one embodiment, the substrate layer is rolled into the shape of a tube.

Electroactive polymers for use in the electroactive polymer actuators of the present invention include polyaniline, polypyrrole, polysulfone and polyacetylene.

In many embodiments, the thrombolysis catheter portion will further comprise one or more structural elements, a specific example of which is a metallic tubular structural element such as a braided wire tube or a laser cut tube.

One advantage of the present invention is that a thrombolysis catheter apparatus can be provided in which the shape of the thrombolysis catheter portion is controlled along a substantial portion of its length. This allows the thrombolysis catheter portion to be efficiently advanced through complex anatomical structures, including hard-to-reach locations in the neurovasculature, allowing thrombolysis procedures to be conducted that would otherwise not be feasible.

Moreover, by controlling the electroactive polymer actuators to impart an orientation to the thrombolysis catheter that is complementary to the three-dimensional spatial trajectory of the blood vessel(s) through which the thrombolysis catheter portion is advanced, the stresses that are placed on the surrounding blood vessel(s) are reduced. This in turn reduces the probability that emboli will be dislodged, for example, from atheroma that are commonly present in cranial blood vessels.

These and other embodiments and advantages of the present invention will become apparent from the following detailed description, and the accompanying drawings, which illustrate by way of example the features of the invention.

### BRIEF DESCRIPTION OF THE DRAWINGS

Fig. 1A is a schematic partial cross-sectional view of a thrombolysis catheter of the prior art;

Fig. 1B is a schematic cross-sectional view of a prior art electroactive polymer actuator useful in connection with the present invention;

Figs. 2-5 are schematic illustrations depicting some possible choices for the deployment of actuators between structural elements, in accordance with various embodiments of the present invention;

Figs. 6A and 6B are schematic perspective views, before and after assembly, of a structural element and a substrate layer with associated components, in accordance with an embodiment of the present invention;

Figs. 6C-6E are schematic cross sectional views illustrating various actuator configurations, in accordance with three embodiments of the present invention;

Fig. 7 is a schematic perspective view of a substrate layer with structural elements incorporated therein, in accordance with an embodiment of the present invention;

Figs. 8A-C are schematic plan views illustrating three orientations of actuators on a substrate, in accordance with various embodiments of the present invention;

FIG. 9 is a schematic perspective view of a thrombolysis catheter in accordance with an embodiment of the present invention;

FIG. 10 is a schematic perspective view of a thrombolysis catheter module, in accordance with the an embodiment of present invention;

FIGS. 11A-C are schematic perspective views illustrating the ability of the thrombolysis catheters of the present invention to retain their orientation at a given depth of insertion;

FIG. 12 is a schematic perspective view of a thrombolysis catheter apparatus, in accordance with an embodiment of the present invention;

FIG. 13 is a schematic perspective view of a thrombolysis catheter apparatus, in accordance with another embodiment of the present invention;

FIG. 14 depicts a thrombolysis catheter apparatus in block diagram format, according to an embodiment of the present invention.

### DETAILED DESCRIPTION OF THE INVENTION

The present invention now will be described more fully hereinafter with reference to the accompanying drawings, in which several embodiments of the present invention are shown. This invention may, however, be embodied in different forms and should not be construed as limited to the embodiments set forth herein.

In many embodiments of the present invention, a thrombolysis catheter is provided in which electroactive polymer actuators are integrated into the thrombolysis catheter structure.

Actuators based on electroactive polymers are preferred for the practice of the present invention due to their small size, large force and strain, low cost and ease of integration into the thrombolysis catheters of the present invention.

Electroactive polymers, members of the family of plastics referred to as "conducting polymers," are a class of polymers characterized by their ability to change shape in response to electrical stimulation. They typically structurally feature a conjugated backbone and have the ability to increase electrical conductivity under oxidation or reduction. Some common electroactive polymers are polyaniline, polysulfone, polypyrrole and polyacetylene. Polypyrrole is pictured below: 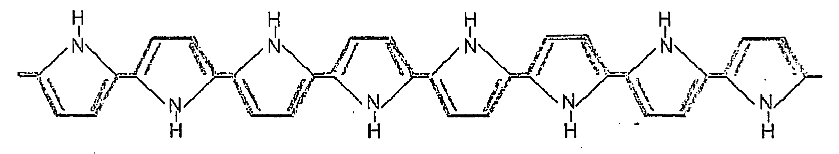 These materials are typically semi-conductors in their pure form. However, upon oxidation or reduction of the polymer, conductivity is increased. The oxidation or reduction leads to a charge imbalance that, in turn, results in a flow of ions into the material in order to balance charge. These ions, or dopants, enter the polymer from an ionically conductive electrolyte medium that is coupled to the polymer surface. The electrolyte may be, for example, a gel, a solid, or a liquid. If ions are already present in the polymer when it is oxidized or reduced, they may exit the polymer.

It is well known that dimensional changes may be effectuated in certain conducting polymers by the mass transfer of ions into or out of the polymer. For example, in some conducting polymers, the expansion is due to ion insertion between chains, whereas in others inter-chain repulsion is the dominant effect. Thus, the mass transfer of ions into and out of the material leads to an expansion or contraction of the polymer.

Currently, linear and volumetric dimensional changes on the order of 25% are possible. The stress arising from the dimensional change can be on the order of 3 MPa, far exceeding that exerted by smooth muscle cells, allowing substantial forces to be exerted by actuators having very small cross-sections. These characteristics are ideal for construction of the thrombolysis catheters of the present invention, as they are small-diameter devices (typically 1 to 5 mm in diameter, more typically 2 to 3 mm) adapted for advancement through the small, tortuous arteries of the neurovasculature.

Referring now to FIG. 1B, taken from U.S. Patent No. 6,249,076, an actuator 10 is shown schematically in cross-section. Active member 12 of actuator 10 has a surface coupled with electrolyte 14 and has an axis 11. Active member 12 includes an electroactive polymer that contracts or expands in response to the flow of ions out of, or into, the active member 12. Ions are provided by electrolyte 14, which adjoins member 12 over at least a portion, and up to the entirety, of the surface of active member 12 in order to allow for the flow of ions between the two media. Many geometries are available for the relative disposition of member 12 and electrolyte 14. In accordance with certain embodiments of the invention, member 12 may be a film, a fiber or a group of fibers, or a combination of multiple films and fibers disposed so as to act in consort for applying a tensile force in a longitudinal direction substantially along axis 11. The fibers may be bundled or distributed within the electrolyte 14.

Active member 12 includes an electroactive polymer. Many electroactive polymers having desirable tensile properties are known to persons of ordinary skill in the art. In accordance with particular embodiments of the invention, active member 12 is a polypyrrole film. Such a polypyrrole film may be synthesized by electrodeposition according to the method described by M. Yamaura et al., "Enhancement of Electrical Conductivity of Polypyrrole Film by Stretching: Counter-ion Effect," Synthetic Metals, vol. 36, pp.209-224 (1988), which is incorporated herein by reference. In addition to polypyrrole, any conducting polymer that exhibits contractile or expansile properties may be used within the scope of the invention. Specific examples include polyaniline, polysulfone and polyacetylene.

Electrolyte 14 may be, for example, a liquid, a gel, or a solid, so long as ion movement is allowed. Moreover, where the electrolyte 14 is a solid, it will typically move with the active member 12 and will typically not be subject to delamination. Where the electrolyte 14 is a gel, it may be, for example, an agar or polymethylmethacrylate (PMMA) gel containing a salt dopant. Where the electrolyte is a liquid, it may be, for example, a phosphate buffer solution. The electrolyte may be non-toxic in the event that a leak inadvertently occurs *in vivo*.

Counter electrode 18 is in electrical contact with electrolyte 14 in order to provide a return path for charge to a source 20 of potential difference between member 12 and electrolyte 14. Counter electrode 18 may be any electrical conductor, for example, another conducting polymer, a conducting polymer gel, or a metal such as gold or platinum, which can be, for example, wire or film form and can be applied, for example, by electroplating, chemical deposition, or printing. In order to activate actuator 10, a current is passed between active member 12 and counter electrode 18, inducing contraction or expansion of member 12. Additionally, the actuator may have a flexible skin for separating the electrolyte from an ambient environment.

The actuators can be provided in an essentially infinite array of configurations as desired, including planar actuator configurations (e.g., with planar active members and counter-electrodes), cylindrical actuator configurations (e.g., see the actuator illustrated in Fig. 1B with cylindrical active member and wire coil counter-electrode), and so forth.

Additional information regarding the construction of actuators, their design considerations, and the materials and components that may be employed therein, can be found, for example, in U.S. Patent No. 6,249,076, assigned to Massachusetts Institute of Technology, and in Proceedings of the SPIE, Vol. 4329 (2001) entitled "Smart Structures and Materials 2001: Electroactive Polymer and Actuator Devices (see, in particular, Madden et al, "Polypyrrole actuators: modeling and performance," at pp. 72-83), both of which are hereby incorporated by reference in their entirety.

As part of a failsafe mechanism for the devices of the present invention, it may be beneficial to select actuators that are of a type that relax in the event that power is interrupted.

Actuators are provided over a substantial portion of the fully inserted length of the thrombolysis catheters of the present invention, for example, typically spanning at least the distal end of the catheter portion, which traverses the tortuous vessels of the neck and head to the site of the occlusion, for example. This is typically the most distal two to six centimeters or so of the thrombolysis catheter, for example, the most distal three centimeters of the catheter. Depending on the location of the occlusion, the actuators can be provided over at least 5%, and in other instances at least 10%, 15%, 25%, 50%, 75%, 90%, or even 100% of the fully inserted length of the thrombolysis catheter portion.

By employing multiple actuators, the thrombolysis catheter portion can be provided with a near infinite range of curvatures, including in-plane curves (e.g., an "S" shaped curve) and out-of-plane curves (e.g., a helix) as well as other far more complex curvatures. For example, the thrombolysis catheter portion can be provided with an out-of-plane curvature that corresponds to the natural orientation of at least a portion of the arterial vasculature, such as the natural orientation of a cranial artery or an internal carotid artery.

The actuators can be disposed within the catheter portion of the present invention in a number of ways. For example, the actuators can be separately manufactured and subsequently attached to structural elements of the catheter portion. As another example, multiple actuators or actuator arrays can be disposed upon a substrate layer, for example, a polymeric sheet, which is intrinsic to the structure of the thrombolysis catheter.

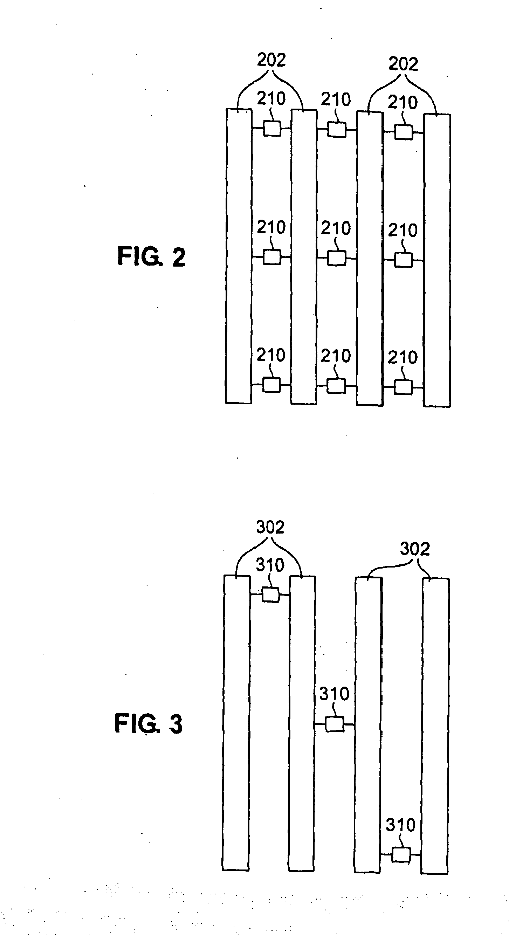

Fig. 2 illustrates one possible configuration of actuators and structural elements in accordance with the present invention, it being understood that the number of actuators and structural elements, as well as the spatial disposition of these elements with respect to one another, can vary widely from one embodiment to another. In the particular embodiment depicted, a series of four annular structural elements 202 are illustrated, with three actuators 210 disposed between each pair of structural elements 202.

While the assembly depicted in Fig. 2 has the actuators disposed along three parallel axes, numerous variations based upon the above noted considerations are possible. For example, the actuators 310 between structural elements 302 can be deployed in a staggered arrangement as illustrated in Fig. 3.

In general, due to their stiffness and elasticity, the thrombolysis catheters of the present invention, are generally inherently biased toward a substantially linear configuration, or other pre-curve shape, in the absence of any applied stress. As a result, the catheter can be bent into any number of configurations by simply contracting one or more of the actuators disposed along its length. Once the actuators are relaxed, the thrombolysis catheter will assume its pre-curve shape (e.g., a more linear configuration).

In alternative designs, multiple actuators can be placed in tension with one another to achieve a desired shape. For example, a series of pivot points can be provided between the structural elements, allowing the catheter to be bent into the desired configuration by placing at least two actuators into tension with one another. Hence, the actuators in a system of this type operate on a principle similar to the operation of skeletal muscles in living organisms such as snakes.

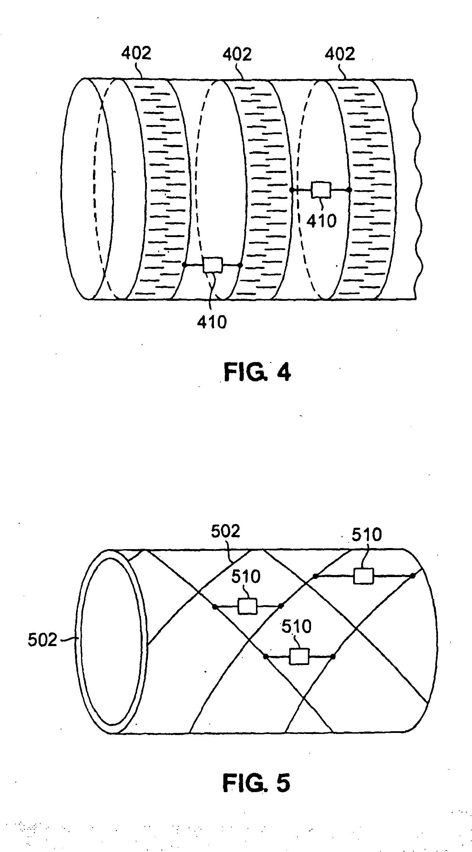

Numerous further variations are possible with respect to structural elements for the catheter portion. For example, while the structural elements are depicted in Figs. 2 and 3 as a series of closed loops, the structural elements can also include open loops, akin to the vertebrae structure of a snake. Moreover, the loops can be replaced by tubes of various lengths if desired. For example, a series of short tubes constructed in a fashion similar to known vascular, biliary or esophageal stents can be used. One such structure is schematically illustrated in Fig. 4, in which actuators 410 are positioned between a series of short stent-like elements 402.

The structural elements may also be combined into a unitary structure, such as a single elongated tube. Thus, the discrete loops in some of the embodiments described above may be replaced, for example, by a helical structural element. The actuators can be deployed between adjacent turns of the helix. In this embodiment, that the adjacent turns of the helix act very much like the series of discrete loops depicted, for example, in Figs. 2 and 3.

Another example of a unitary structure is illustrated in Fig. 5, which incorporates a stent-like mesh structure 502. Referring to Fig. 5, actuators 510 are disposed between adjacent members of mesh structure 502. The mesh structure 502 is typically flexible and elastic such that it possesses an inherent bias or memory that acts to restore the assembly to its original (e.g., substantially linear) configuration. Moreover, in the final catheter structure, the mesh structure illustrated will typically have an inner liner and an outer jacket, either or both of which may be elastic in nature, biasing the catheter, for example, towards a substantially linear configuration. The actuators 502 can then be used to deflect the structure from this configuration as needed.

In general, the shape of the catheter portion of the present invention can be inferred from the intrinsic position-dependent electrical properties of the electroactive polymer actuators. However, if desired, a number of strain gauges can be employed to provide electronic feedback concerning the orientation of the actuators and structural elements within the assembly. This electronic feedback will also provide a number of additional advantages, including compensation for physiologic changes, greater stability, error correction, and immunity from drift. Strain gauges suitable for use in the present invention include (a) feedback electroactive polymer elements whose impedance or resistance varies as a function of the amount of strain in the device and (b) conventional strain gauges in which the resistance of the device varies as a function of the amount of strain in the device, thus allowing the amount of strain to be readily quantified and monitored. Such strain gauges are commercially available from a number of different sources, including National Instruments Co., Austin, TX, and include piezoresistive strain gauges (for which resistance varies nonlinearly with strain) and bonded metallic strain gauges (for which resistance typically varies linearly with strain).

Feedback regarding the shape of the catheter portion, as well as the relationship between the catheter portion and the lumen into which it is inserted, may also be readily obtained using medical diagnostic imaging data generated, for example, from diagnostic angiograms, sonograms, CT or MR scans, IVUS data, or fluoroscopic images (which may be multiplane or tomographic). If desired, the catheter portion can be provided with opaque markers, e.g., radio-opaque markers, to provide more precise feedback regarding the shape and position of the catheter portion.

As another example, electromagnetic position sensors may be included in the thrombolysis catheter structure to provide an electronic readout of the 3D shape and position of the thrombolysis catheter, which is independent of medical diagnostic imaging data. Such electromagnetic position sensors have been used in animation and metrology, and are presently emerging in cardiology and electrophysiology. Examples of such systems are the NOGA^{™} cardiology navigation system and the CARTO^{™} electrophysiology navigation system, both available from Biosense Webster, Diamond Bar, CA, as well as the RPM Realtime Position Management^{™} electrophysiology navigation system available from Boston Scientific Corporation, Natick, MA.

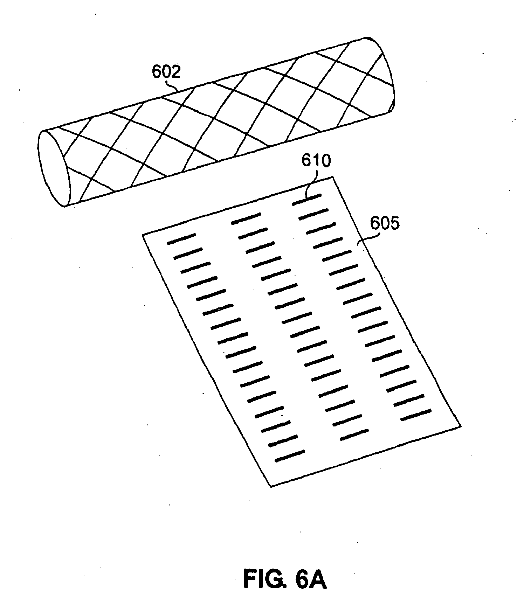

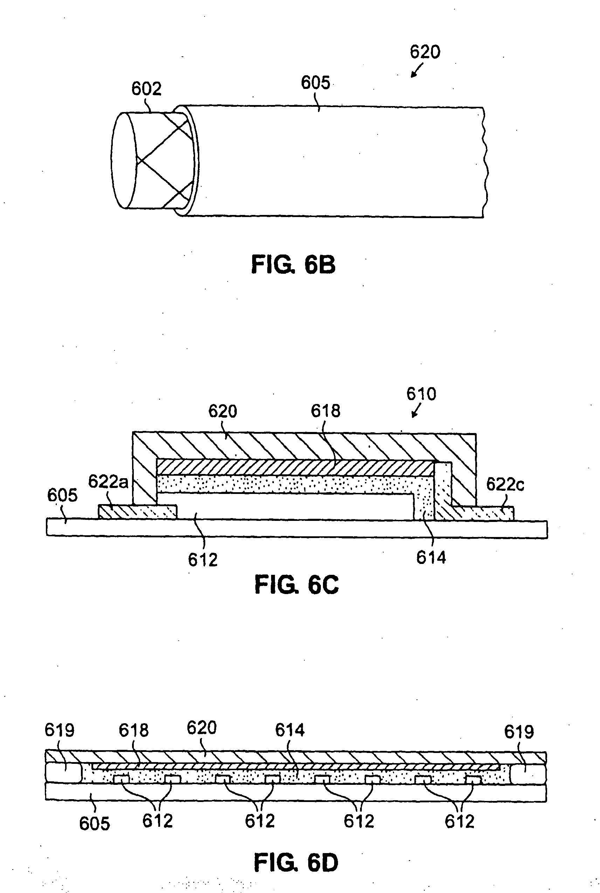

In the embodiments described above, the actuators are directly coupled to the structural elements of the thrombolysis catheter portion. However, this need not be the case as illustrated, for example, in Figs. 6A and 6B. Fig. 6A illustrates a structural element 602, which consists of a braided wire tube, as well as a flexible substrate layer 605. A series of actuators 610 (a single actuator is numbered) is printed on substrate layer 605, along with a control bus (not shown) for transmitting control signals to the actuators 610 from a controlling device.

The substrate layer 605 is then wrapped around a structural element 602, and the edges are joined (or overlapped), forming a tubular substrate layer and providing the cylindrical assembly 620 illustrated in Fig. 6B. In this design, the structural element 602 (and in many cases the substrate layer 605) will act to bias the overall assembly 620 toward a pre-curve configuration, which can be, for example, a linear configuration. The actuators 610 are used to deflect this structure to the desired degree.

In some embodiments, and to the extent that substrate layer 605 is not lubricious, it may be desirable to dispose a lubricious outer jacket (e.g., a hydrogel coating, a silicone, or a fluoropolymers) over the assembly to facilitate advancement of the thrombolysis catheter.

A number of flexible tubular structural elements are known besides the structural element 602 of Fig. 6A-B, which can be employed in the present invention. For example, numerous flexible tubular structural elements are known from the stent art, including vascular, biliary or esophageal stents. These tubular constructions are typically metal, and include (a) tubular open-mesh networks comprising one or more knitted, woven or braided metallic filaments; (b) tubular interconnected networks of articulable segments; (c) coiled or helical structures (including multiple helices) comprising one or more metallic filaments; (d) patterned tubular metallic sheets (e.g., laser-cut tubes), and so forth.

In addition, catheter configurations consisting of an inner liner and an outer jacket, with a flexible tubular structural element (typically metallic, for example, a tube formed from braided or helical stainless-steel wire or a cut stainless steel tube) disposed between the inner liner and outer jacket are known for example, from the guide catheter art. Such structures can be readily adapted to achieve the purposes of the present invention.

Referring once again to Figs. 6A and 6B, the substrate layer 605 that is employed in these figures can be selected from a number of flexible materials, and is typically formed from one or more polymeric materials. Polymeric materials useful in the construction of the substrate layer 605 include the following polymeric materials: polyolefins such as metallocene catalyzed polyethylenes, polypropylenes, and polybutylenes and copolymers thereof; ethylenic polymers such as polystyrene; ethylenic copolymers such as ethylene vinyl acetate (EVA), butadiene-styrene copolymers and copolymers of ethylene with acrylic acid or methacrylic acid; polyacetals; chloropolymers such as polyvinylchloride (PVC); fluoropolymers such as polytetrafluoroethylene (PTFE); polyesters such as polyethylene terephthalate (PET); polyester-ethers; polysulfones; polyamides such as nylon 6 and nylon 6,6; polyamide ethers such as polyether block amides; polyethers; elastomers such as elastomeric polyurethanes and polyurethane copolymers; silicones; polycarbonates; polychloroprene; nitrile rubber; butyl rubber; polysulfide rubber; *cis*-1,4-polyisoprene; ethylene propylene terpolymers; as well as mixtures and block or random copolymers of any of the foregoing are examples of biostable polymers useful for manufacturing the medical devices of the present invention.

In some embodiments, the substrate layers are constructed from stiff polymers like those used in electronic printed circuits or cables, such as polyimide (e.g., Kapton®), and relieved by selective cutting, e.g. with a laser, to provide the appropriate flexibility.

Inner and/or outer jacket materials for the thrombolysis portion can also be selected form the above polymers, where desired.

Although Fig. 6A illustrates a single substrate layer 605, multiple substrate layers can be used. For example, an additional substrate layer can be provided which contains a plurality of strain gauges, for example, feedback polymer elements, along with a readout bus for transmitting information from the strain gauges to a controlling device.

Actuators 610 can be provided on substrate layer 605 in numerous configurations. For example, a single actuator 610 is shown in cross-section in Fig. 6C, disposed on substrate layer 605. As previously discussed, the actuator 610 typically includes an active member 612 and counter-electrode 618, with an intervening electrolyte-containing layer 614.

As also previously discussed, the active member 612 preferably comprises an electroactive polymer, many of which are known in the art. Polypyrrole, polysulfone, polyacetylene and polyaniline are specific examples. The counter-electrode 618 may be any suitable electrical conductor, for example, another conducting polymer, a conducting polymer gel, or a metal such as gold or platinum, typically in a flexible form, for example, in the form of a thin layer or foil. The electrolyte within the electrolyte-containing layer 614 can be, for example, a liquid, a gel, or a solid as previously discussed.

It is beneficial that the active members 612 avoid contact with the counter-electrode 618 to prevent short-circuiting. In the embodiment illustrated, such contact is prevented by provided the electrolyte within a flexible porous layer of insulating polymer material. Beneficial insulating polymers for this purpose include insulating polymers within the polymer list that is provided above in connection the substrate layer 605. PTFE is a specific example.

Track wires 622a and 622c are connected to active member 612 and counter-electrode 618, respectively, allowing for electrical communication with a controlling device (not shown).

A barrier layer 620 may be provided for several reasons. For example, the barrier layer 620 can prevent species within the electrolyte-containing layer 614 from escaping. Appropriate materials for the barrier layer include those discussed above in connection with substrate layer 605.

Numerous actuator configurations other than that illustrated in Fig. 6C are also possible. For example, Fig. 6D is a cross-section illustrating eight active members 612 disposed on substrate layer 605. Over the active members 612 are electrolyte-containing layer 614, counter-electrode layer 618 and barrier layer 620. The barrier layer 620 is sealed to the substrate layer 605 using, for example, an adhesive 619. The configuration of Fig. 6D contains a common counter-electrode 618. The active members 612 are typically provided with discrete track wires (not shown) for individual activation.

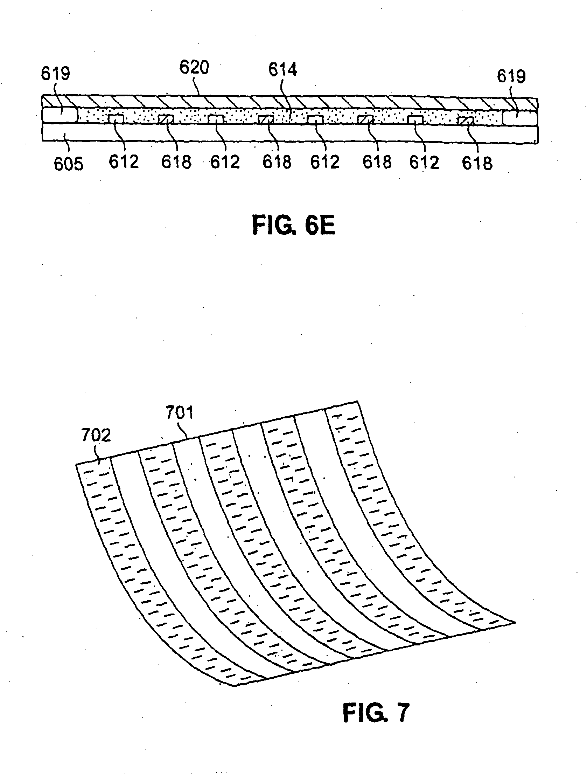

As another example, Fig. 6E is a cross-section including five active members 612 disposed and four counter-electrode regions 618 disposed on a substrate layer 605. An electrolyte-containing layer 614 contacts the active members 612 and counter-electrode regions 618. A barrier layer 620 is sealed to the substrate layer 605 using, for example, an adhesive 619. The active regions are typically provided with discrete track wires (not shown) for individual activation. The counter-electrode regions 618 can also be provided with discrete track wires (not shown), or these regions can constitute portions of a single counter-electrode (e.g., a digitated structure).

If desired, structural elements for the thrombolysis catheter portion can also be provided on a substrate layer. For example, Fig. 7 illustrates substrate layer 701 having printed thereon a series of relatively stiff structural elements 702 which, when rolled up, will form structural elements similar to those illustrated in Fig. 4.

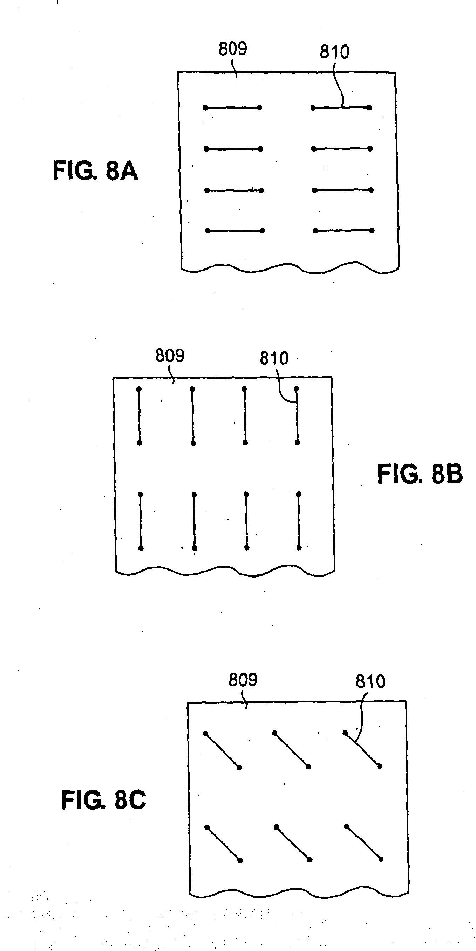

Although the actuators illustrated in the above figures are oriented in the direction of the thrombolysis catheter axis, the actuators can be oriented in essentially any direction desired for control. For example, Figs. 8A, 8B and 8C illustrate three substrate layers 809, each having a series of actuators 810 (one actuator is numbered in each figure), which are oriented in various directions. By laminating these substrate layers together, a composite structure (not shown) can be created which can bend, contract circumferentially, and so forth.

If desired, the thrombolysis catheter of the present invention can be stiffened during use. The catheter can be stiffened all along its length or only over a portion of its length (e.g., at the distal end) in accordance with the invention. The stiffness of the thrombolysis catheter can be adjusted in a number of ways. As one example, actuators can be disposed within the thrombolysis catheter such that they are in tension with one another as discussed above (e.g., in a fashion analogous to skeletal muscles). Such a thrombolysis catheter can be stiffened by placing opposing actuators into tension with one another.

Each actuator within the thrombolysis catheters of the present invention may be individually controllable. This allows these elements to be driven for the purpose of effecting changes to the configuration of the overall device. For example, the actuators (and strain gauges, if desired) may be placed in direct communication with a controlling device by means of dedicated circuits linking each of these elements to the device. However, it is more typical to deploy these elements such that each element is in communication with the controlling device by means of a common communications cable. The signals from each element may be digital or analog. If need be, digital-to-analog or analog-to-digital converters may be provided to convert the signals from one format to the other.

The signals to and from each element may be conveniently managed and transmitted over a common cable by multiplexing. Multiplexing schemes that may be used for this purpose include frequency-division multiplexing, wave-division multiplexing, or time-division multiplexing. Suitable multiplexers and demultiplexers can be employed at each end of the cable and along its length at the position of each actuator or gage.

In terms of electronic data storage, each actuator (and strain gauge, if desired) may be given a separate address in electronic memory where information concerning the state of the element is stored. This information may be accessed to determine the state of the device, or for the purpose of performing operations on the device or its elements. The memory in which the information is stored may be of a volatile or non-volatile type, and may be in the device itself, but is typically in a separate control and display device (e.g., a personal computer, such as a laptop computer).

Numerous cable configurations are possible. For example, cables can be directly connected to the actuators. Alternatively, the cables can be printed onto a substrate layer (see, e.g., track wires 622a, 622c illustrated in Fig. 6C). In this case, each substrate layer upon which the actuators (and strain gauges, if desired) are disposed may be similar to a flexible printed circuit board in that the necessary elements are printed upon a flexible substrate. Each layer can be provided with its own track wires and communication cables (e.g., the control, and readout buses discussed above). As an alternative, the actuators (and strain gauges, if desired) can be connected to a separate interconnect layer, for example, by plated through-holes or vias (these also can function as "rivets" to hold the stack of sheets together). Such through-holes can tie into a series of conductive track wires disposed on the interconnect layer, which track wires can connect to a "spinal cord", such as a cable bundle, flat cable or ribbon cable that runs the length of the device.

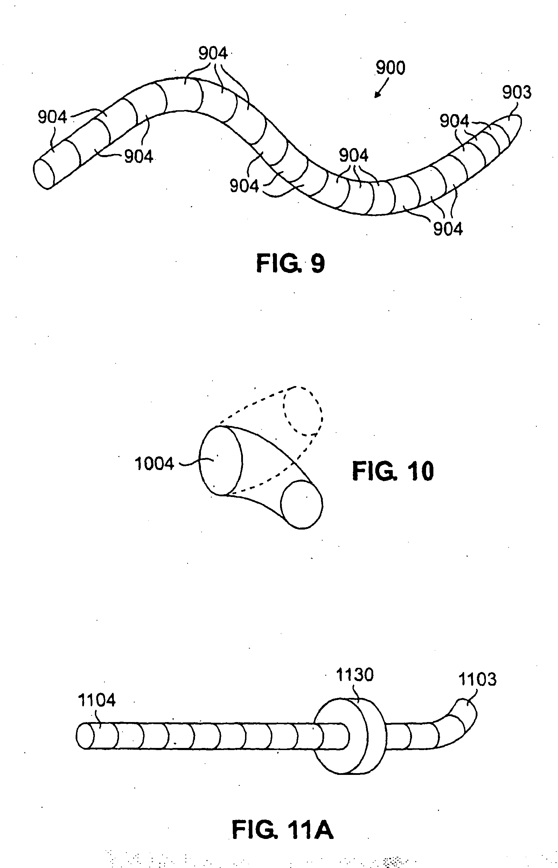

In some embodiments, the thrombolysis catheters of the present invention are divided into a series of "deflection modules", each of which includes a plurality of actuators that allow the module to take on a variety of shapes in 3-dimensional space in response to input by a control device. The greater the number of modules, the finer the control of the 3-dimensional orientation of the thrombolysis catheter portion. A simplified schematic diagram of a thrombolysis catheter 900 with eighteen modules 904 and a tip 903 (e.g., a soft tip to reduce risk of trauma during catheter advancement) is illustrated in Fig. 9. The overall shape of the thrombolysis catheter is established by manipulating the deflection of each of the modules. For example, as illustrated in Fig. 10, the actuators can be activated to deflect a given module 1004 from a first position (designated by solid lines) to a second position (designated by dashed lines). Additional degrees of freedom in deflection are also possible, e.g., changes in diameter or changes in length.

In use, the thrombolysis catheter is typically advanced through a valved introducer fitting, up the arteries of the arm or leg of the patient (which can be, for example, a vertebrate animal, and preferably a human), through the aorta and to a desired artery. For example, the catheter can be advanced to an occlusion in the middle cerebral artery via the aorta, common carotid artery and internal carotid artery. Of course, occlusions can occur essentially anywhere in the neurovasculature and include cerebral artery occlusions (for example, middle cerebral artery occlusions, which are most common, as well as posterior cerebral artery occlusions and anterior cerebral artery occlusions), internal carotid artery occlusions, and basilar artery occlusions.

Once the thrombolysis catheter reaches its target location (for example, an occlusion in the neurovasculature), an appropriate thrombolysis procedure is performed. For example, a thrombolytic agent such as heparin or urokinase can be delivered from the catheter, or a non-chemical procedure can be employed such as an angioplasty procedure, an elevated temperature thrombolysis procedure (e.g., a laser thrombolysis procedure) or a mechanical thrombolysis procedure (e.g., a hydraulic thrombolysis or ultrasound thrombolysis procedure). One desirable technique is a laser thrombolysis technique such as that discussed above in connection with Fig. 1A.

In some embodiments, the thrombolysis catheter is provided with a steering system, which is used to control electronic actuators in the thrombolysis catheter tip. A number of options are available for catheter steering. For example, the thrombolysis catheter can be provided with a manual steering system that is operated under image guidance. Electrical control from the control unit can be based, for example, on manual steering input using a joystick or the like.

Image guidance can be obtained using a number of techniques. For example, image guidance can be obtained from a medical diagnostic imaging data such as that discussed above. If desired, the catheter portion can be provided with opaque markers, such as radio-opaque markers, to improve image definition.

Multiple other techniques can also be used to provide image guidance. For example, an image of the body lumen into which the catheter portion is inserted can be obtained using medical diagnostic imaging data, while an image of the catheter portion within the lumen can be obtained by providing electromagnetic sensors such as those discussed above within the catheter portion.

Steering control can also be automated. For example, based on inputted medical diagnostic imaging data and/or electromagnetic sensor data, actuator control can be provided by means of an edge-tracking or center-seeking algorithm to keep the distal end of the thrombolysis catheter at or near the center of the body lumen.

In still other embodiments, the thrombolysis catheter is steered in a semiautomatic fashion, for example, using a computer algorithm like that discussed above to suggest a direction of travel, with a trained operator acting to either accept or reject the computer-generated suggestion. In this instance, it may be desirable to tailor the algorithm to reflect operator preferences based upon operator profiles.

In some embodiments, the thrombolysis catheter system is provided with a shape changing system, which is used to control electronic actuators along the thrombolysis catheter length during the insertion process. Numerous options are available.

For example, in certain embodiments of the invention, the overall shape of the thrombolysis catheter portion is modified based upon information regarding the configuration of the catheter portion, including the relationship between the catheter portion and the body lumen into which it is inserted. For example, information regarding the spatial orientation of the catheter portion can be obtained via electromagnetic sensors within the catheter portion or from strain gauges along the length of the thrombolysis catheter, while information regarding the spatial orientation of the body lumen into which the thrombolysis catheter is inserted can be obtained using medical diagnostic imaging data.

This combined information can be used to control, and provide feedback regarding, the overall shape of the thrombolysis catheter portion.

For example, the above data can be used to construct a virtual image of the catheter and blood vessel of interest on a display associated with the controlling device (e.g., on the screen of a laptop computer). Based on this information, an operator can determine a desired shape change for the thrombolysis catheter, which can be input into the control unit, for example, by using a mouse to move virtual onscreen catheter elements to a desired configuration. Subsequently, the control unit drives the actuators within the thrombolysis catheter to achieve this desired configuration.

In other embodiments, as the thrombolysis catheter is advanced into a body lumen, a 3-dimensional representation the desired shape of the thrombolysis catheter can be stored into memory, with further data being added with increasing depth of insertion.

For example, the orientation of the thrombolysis catheter tip (herein referred to as a "lead module") as a function of position can be stored within a computer, acting as a map for subsequent deflection modules.

Position data can be provided, for example, from a depth gauge or linear displacement transducer placed at the site of thrombolysis catheter introduction. As one specific example, a depth gauge can be supplied, which contains a rotating gear wheel whose revolutions are monitored. As other examples, a linear displacement transducer containing a depth code which can be read optically (using, for example, bar-codes and an optical source and detector) or magnetically (using, for example, a magnetic code and a Hall effect sensor) can be used to determine the extent of thrombolysis catheter advancement. Alternatively, position data can be provided by placing electromagnetic position sensors within the catheter portion as discussed above. These and numerous other known methods are available for determining position.

The data relating to the orientation of the lead module can be provided, for example, using input from a steering step (e.g., input from a joystick or input from a edge or center-seeking computer algorithm), from strain gauges within the lead module, or from electromagnetic position sensors within the lead module (assuming a sufficient number are present to provide adequate resolution).

Using this position and orientation information, electrical control signals for the actuators are calculated as a function of position. As subsequent modules arrive at the position that was previously occupied by the lead module, the actuators within these subsequent modules are operated such that they assume the orientation of the lead module when it was present at that particular depth of insertion.

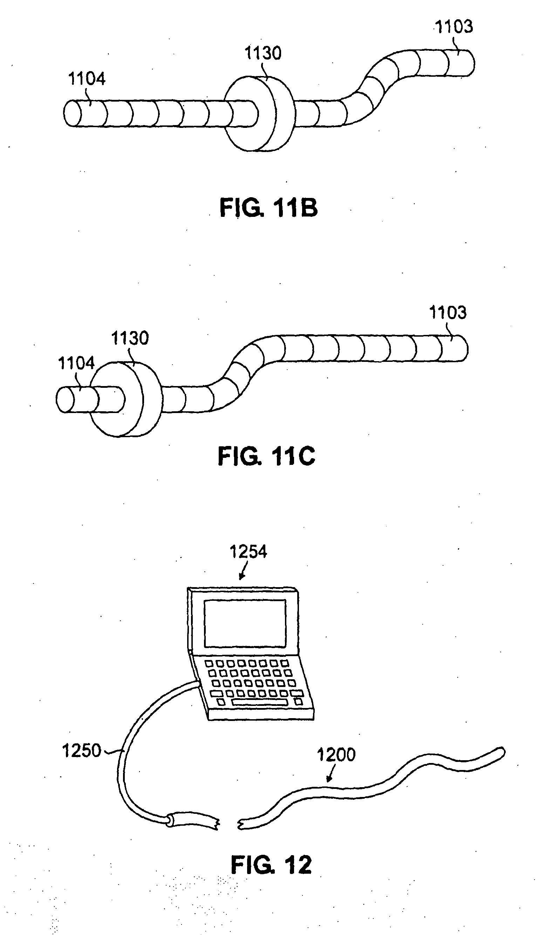

The result of the above is that the thrombolysis catheter retains its path in 3-dimensional space, reflecting the shape of the lumen that it travels through. This is illustrated in Figs. 11A-C, which contain simplified schematic diagrams of a thrombolysis catheter, consisting of a number of deflection modules 1104 (one numbered) and a lead module 1103, as well as a linear displacement transducer 1130. These figures illustrate the orientation of the thrombolysis catheter: shortly after insertion (Fig. 11A); at an intermediate point of insertion (Fig. 11B); and at a point of full insertion (Fig. 11C). As seen from these figures, as it advances, the thrombolysis catheter retains its orientation at a given depth of insertion.

Fig. 12 is a simplified schematic diagram of a thrombolysis catheter apparatus in accordance with an embodiment of the invention. The thrombolysis catheter apparatus includes a thrombolysis catheter portion 1200 containing numerous electronic actuators (not shown) that are controlled by a control unit, such as a computer 1254. An electronic cable bundle 1250 is provided between the thrombolysis catheter portion 1200 and an electronic interface, including drivers, which is provided within the computer 1254. Signals are sent from drivers in the electronic interface through cable bundle 1250 to the actuators within the thrombolysis catheter portion 1200, controlling the three dimensional shape of the thrombolysis catheter portion 1200. If desired, a steering mechanism, such as a computer mouse pad or a built-in or peripheral joystick, may be used to steer and control the thrombolysis catheter portion 1200 as discussed above. In some embodiments of the invention, the thrombolysis catheter portion 1200 is provided with strain gauges, in which case signals are output from the strain gauges and sent via the cable bundle 1250 to the electronic interface within the computer 1254. These signals are processed within the computer 1254, for example, to (a) provide the actuators with stability, error correction, and immunity from drift and (b) provide an a virtual image of the thrombolysis catheter orientation *in vivo*, if desired.

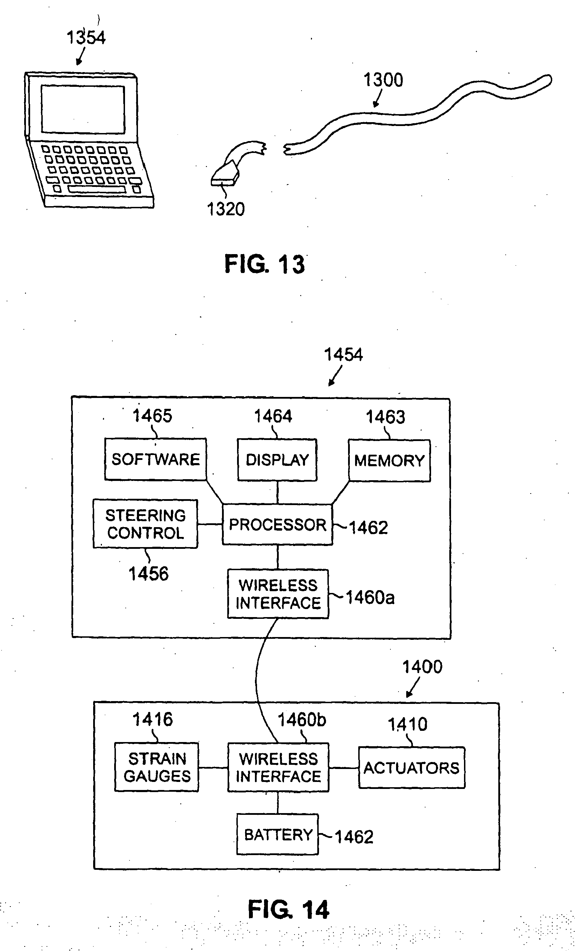

A wireless alternative to the embodiment of Fig. 12 is illustrated in Fig. 13. The thrombolysis catheter apparatus illustrated in Fig. 13 includes a thrombolysis catheter portion 1300 containing numerous electronic actuators (not shown) that are controlled by a control unit, such as a computer 1354. A power source (not shown) and a wireless interface including drivers (not shown) are provided within the proximal end of the thrombolysis catheter portion 1300. The wireless interface of the thrombolysis catheter portion 1300 communicates with a companion wireless interface within a remote computer 1354.

The thrombolysis catheter apparatus of Fig. 13 beneficially utilizes wireless interface chipsets, which can be less expensive and more reliable than electrical connectors such as the cable bundle 1250 of Fig. 12. Inexpensive wireless interfaces are presently available from a number of sources, including Bluetooth^{™} wireless interfaces available from Motorola and IEEE 802.11b wireless interfaces available, for example, from Cisco, Apple and Lucent. Depending on the economics, multiple wireless interfaces can be provided, for example, one for each module of the thrombolysis catheter.

The power source for the thrombolysis catheter portion 1300 is typically a battery. By building battery power into the thrombolysis catheter portion 1300, interconnection cost and complexity are reduced. One or more batteries can be provided essentially anywhere within the thrombolysis catheter portion, and are beneficially provided at the proximal end of the thrombolysis catheter portion 1300, which can be, for example, in the form of an integrated, sealed control handle 1320. The electronics for the wireless interface, including drivers for the electronic actuators and other components, are also beneficially provided at the proximal end of the thrombolysis catheter portion 1300.

One embodiment of a thrombolysis catheter apparatus of the present invention is presented in block diagram format in FIG. 14. The thrombolysis catheter apparatus shown includes a thrombolysis catheter portion 1400 and a computer 1454. The thrombolysis catheter portion 1400 is powered by battery 1423. A wireless interface 1460a and 1460b (including drivers) is provided between the thrombolysis catheter portion 1400 and the computer 1454. Control signals for the actuators 1410 within the thrombolysis catheter portion 1400 are sent from the computer 1454 to the thrombolysis catheter portion 1400 via the wireless interface 1460a, 1460b. At the same time, data (e.g., data from the strain gauges 1416) is sent from the thrombolysis catheter portion 1400 to the computer 1454 via the wireless interface 1460a, 1460b.

As is typical, the computer 1454 contains a processor 1462, memory 1463 and display 1464. If desired, strain gauge data transmitted over the wireless interface 1460a, 1460b can be processed by software 1465 to present a virtual image of the thrombolysis catheter portion 1400 on the display 1464 (as an alternative example, a medical diagnostic image, for example, an angiogram or an image generated from electromagnetic sensors in the catheter portion, can be presented on the display 1464). The operator can change the configuration of the thrombolysis catheter portion 1400, for example, by operating the steering control 1456 to provide an input signal that is used by the operating software 1465 (along with any other input signals, such as data from strain gauges, electromagnetic sensors, etc.) to calculate a control signal. The control signal is sent to the actuators 1410 in the thrombolysis catheter portion 1400 via drivers in the wireless interface 1460b to steer and control the shape of the thrombolysis catheter portion 1400.

Although the present invention has been described with respect to several exemplary embodiments, there are many other variations of the above-described embodiments that will be apparent to those skilled in the art, even where elements have not explicitly been designated as exemplary. It is understood that these modifications are within the teaching of the present invention, which is to be limited only by the claims appended hereto.

### Examples of catheters are listed below:

1. A thrombolysis catheter apparatus, comprising:
   (a) an elongated thrombolysis catheter portion comprising a plurality of independently controllable electroactive polymer actuators, said actuators providing a curvature for said thrombolysis catheter portion based upon received control signals;
   (b) a control unit coupled to said plurality of actuators and sending said control signals to said plurality of actuators; and
   (c) an occlusion removal device.
2. The thrombolysis catheter apparatus of number 1, wherein said occlusion removal device comprises a laser and a light guide, said laser being optically coupled to said light guide.
3. The thrombolysis catheter apparatus of number 1, wherein said plurality of electroactive polymer actuators are disposed along at least 2 cm of the axial length of the thrombolysis catheter portion.
4. The thrombolysis catheter apparatus of number 1, wherein at said thrombolysis catheter portion comprises at least three independently controllable electroactive polymer actuators.
5. The thrombolysis catheter apparatus of number 1, wherein said actuators are disposed within said thrombolysis catheter portion such that said thrombolysis catheter portion is provided with a shape that comprises an out-of-plane curve.
6. The thrombolysis catheter apparatus of number 5, wherein said out-of-plane curve corresponds to a natural orientation of at least a portion of a cranial or cerebral artery.
7. The thrombolysis catheter apparatus of number 5, wherein said out-of-plane curve corresponds to a natural orientation of at least a portion of an internal carotid artery.

## Claims

1. A thrombolysis catheter apparatus, comprising:
• an elongated thrombolysis catheter portion comprising a plurality of independently controllable electroactive polymer actuators, provided on substrate layer (605) made from a flexible material and wrapped into a cylinder, said actuators providing a curvature for said thrombolysis catheter portion based upon received control signals;
• a control unit coupled to said plurality of actuators and sending said control signals to said plurality of actuators; and
• an occlusion removal device.

2. The thrombolysis catheter apparatus of claim 1, wherein said occlusion removal device comprises a laser and a light guide, said laser being optically coupled to said light guide.

3. The thrombolysis catheter apparatus of claim 1, wherein said plurality of electroactive polymer actuators are disposed along at least 2 cm of the axial length of the thrombolysis catheter portion.

4. The thrombolysis catheter apparatus of claim 1, wherein at said thrombolysis catheter portion comprises at least three independently controllable electroactive polymer actuators.

5. The thrombolysis catheter apparatus of claim 1, wherein said actuators are disposed within said thrombolysis catheter portion such that said thrombolysis catheter portion is provided with a shape that comprises an out-of-plane curve.

6. The thrombolysis catheter apparatus of claim 5, wherein said out-of-plane curve corresponds to a natural orientation of at least a portion of a cranial or cerebral artery.

7. The thrombolysis catheter apparatus of claim 5, wherein said out-of-plane curve corresponds to a natural orientation of at least a portion of an internal carotid artery.
